# EUROPEAN PATENT APPLICATION

(11) **EP 1 698 339 A1**
(43) Date of publication of application: **06.09.2006**
(21) Application number: 04807556.8
(22) Date of filing: 22.12.2004
(51) Int. Cl.: A61K 31/501, A61P 7/00, A61P 11/00, C07D 401/12

(54) **NEUTROPHILIA INHIBITOR**

(30) Priority: 26.12.2003 JP 2003433747
(71) Applicant: Nissan Chemical Industries, Ltd., Chiyoda-ku, Tokyo 101-0054 (JP); TAISHO PHARMACEUTICAL CO., LTD, Tokyo 170-8633 (JP)
(72) Inventor: IWAMA, Takehisa, Nissan Chemical Industries Ltd, Minamisaitama-gun, Saitama 3490218 (JP); TSURUZOE, Nobutomo, Nissan Chemical Industries Ltd, Minamisaitama-gun, Saitama 3490218 (JP)
(74) Representative: Blodig, Wolfgang
(86) International application number: PCT/JP2004/019199
(87) International publication number: WO 2005/063250

(57) **Abstract**

To provide an antineutrophilia agent effective for treatment of development and progress of acute infections, collagen diseases (chronic rheumatoid arthritis, Wegener's granulomatosis and Behcet's disease), chronic obstructive pulmonary disease (COPD), chronic bronchitis, pulmonary emphysema, small airway disease, gout, Cushing's syndrome, myelofibrosis, neoplastic neutrophilia, polycythemia vera and diseases caused by administration of steroid drugs.

An antineutrophilia agent containing a 3(2H)-pyridazinone compound represented by the formula (I) or a pharmaceutically acceptable salt thereof [wherein each of R¹, R² and R³ is independently a hydrogen atom or a C₁₋₆ alkyl group, X is a halogen atom, cyano or a hydrogen atom, Y is a halogen atom, trifluoromethyl or a hydrogen atom, and A is a C₁₋₈ alkylene which may be substituted with a hydroxyl group].

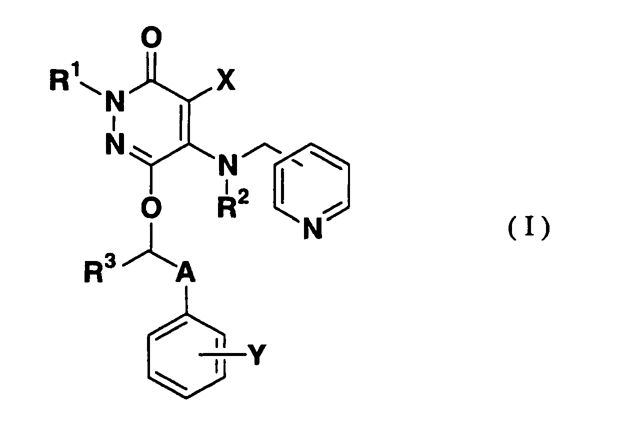

## Description

### TECHNICAL FIELD

The present invention relates to an antineutrophilia agent containing a pyridazinone compound or a pharmaceutically acceptable salt thereof as an active ingredient.

### BACKGROUND ART

Highly safe drugs which selectively reduce neutrophils are promising as preventive or therapeutic agents for some diseases for which few drugs are medically useful at present. Namely, use of neutrophilia is expected for diseases accompanied by abnormally high neutrophil counts. As specific examples, diseases which develop and progress with involvement of neutrophils such as acute infections (bacterial, fungal, spirochete, parasitic, rickettsial and viral infections), collagen diseases (chronic rheumatoid arthritis, Wegener's granulomatosis and Behcet's disease), chronic obstructive pulmonary disease (COPD), chronic bronchitis, pulmonary emphysema, small airway disease, gout, Cushing's syndrome, myelofibrosis, neoplastic neutrophilia, polycythemia vera and diseases caused by administration of steroid drugs may be mentioned.

Pyridazinone compounds or their salts are known to have excellent antithrombotic action, cardiotonic action, vasodilator action, anti-SRS-A (Slow Reacting Substance of Anaphylaxis) action, thromboxane A2 synthetase inhibitory action, therapeutic action on spinal canal stenosis and erectile dysfunction and angiogenesis stimulatory and enhancing actions and are promising as antiplatelet agents (Patent Documents 1 to 6).

However, it has not been known what effect these pyridazinone compounds have on neutrophilia. On the other hand, though among various treatments for neutrophilia, chemotherapy is an established one, a better chemotherapy is demanded.
Patent Document 1: JP-B-7-107055
Patent Document 2: JP-A-7-252237
Patent Document 3: JP-A-7-285869
Patent Document 4: WO99/11268
Patent Document 5: WO00/12091
Patent Document 6: WO00/33845

### DISCLOSURE OF THE INVENTION

### PROBLEMS THAT THE INVENTION IS TO SOLVE

The object of the present invention is to provide an excellent antineutrophilia agent.

### MEANS OF SOLVING THE PROBLEMS

As a result of their extensive research, the present inventors have found that the pyridazinone compounds represented by the following formula (I) or their pharmaceutically acceptable salts have excellent antineutrophilia effect and have accomplished the present invention.

Namely, the present invention provides:
(1) An antineutrophilia agent containing a 3(2H)-pyridazinone compound represented by the formula (I) or a pharmaceutically acceptable salt thereof: [ka 1] 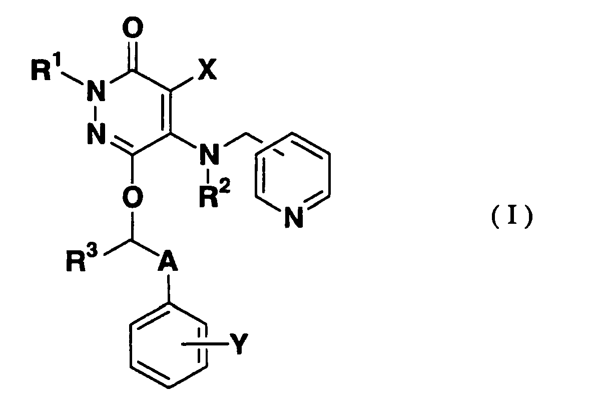 [wherein each of R¹, R² and R³ is independently a hydrogen atom or a C₁₋₆ alkyl group, X is a halogen atom, cyano or a hydrogen atom, Y is a halogen atom, trifluoromethyl or a hydrogen atom, and A is a C₁₋₈ alkylene which may be substituted with a hydroxyl group].
(2) The antineutrophilia agent according to (1), wherein in the formula (I), R¹ and R² are hydrogen atoms, R³ is a hydrogen atom or a C₁₋₄ alkyl group, X is a halogen atom, Y is a halogen atom or a hydrogen atom, and A is a C₁₋₅ alkylene which may be substituted with a hydroxyl group.
(3) The antineutrophilia agent according to (1), wherein the compound represented by the formula (I) is 4-bromo-6-[3-(4-chlorophenyl)propoxy-5-(3-pyridylmethylamino)-3(2H)-pyridazinone or 4-bromo-6-[3-(4-chlorophenyl)-3-hydroxypropoxy]-5-(3-pyridylmethylamino)-3(2H)-pyridazinone.
(4) The antineutrophilia agent according to (1), wherein the pharmaceutically acceptable salt is an organic acid salt or an inorganic acid salt.
(5) The antineutrophilia agent according to any one of (1) to (4), which is a preventive or therapeutic agent for chronic obstructive pulmonary disease.

The antineutrophilia agent of the present invention is preferably a pyridazinone compound of the formula (I) wherein R¹ and R² are hydrogen atoms, R³ is a hydrogen atom or a C₁₋₄ alkyl group, X is a halogen atom, Y is a halogen atom or a hydrogen atom, and A is a C₁₋₅ alkylene which may be substituted with a hydroxyl group, or a pharmaceutically acceptable salt thereof.

The pyridazinone compound represented by the formula (I) in the antineutrophilia agent of the present invention is particularly preferably 4-bromo-6-[3-(4-chlorophenyl)propoxy-5-(3-pyridylmethylamino)-3(2H)-pyridazinone or 4-bromo-6-[3-(4-chlorophenyl)-3-hydroxypropoxy]-5-(3-pyridylmethylamino)-3(2H)-pyridazinone.

### EFFECTS OF THE INVENTION

The present invention provides a novel antineutrophilia agent containing a pyridazinone compound (I) or a pharmaceutically acceptable salt thereof.

### BRIEF DESCRIPTION OF THE DRAWING

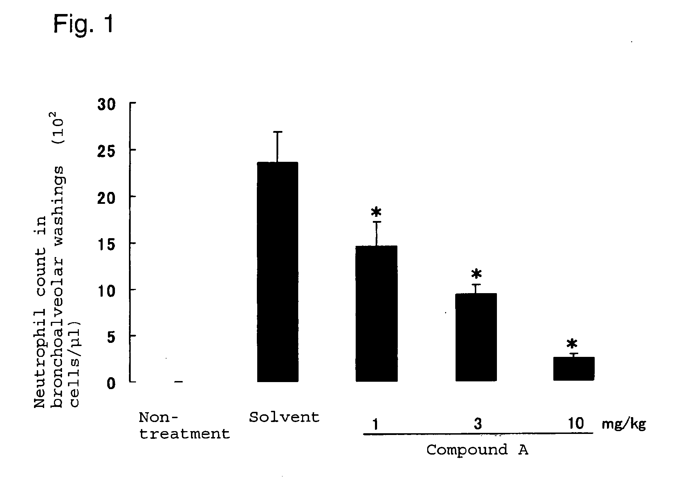

[Fig. 1] Fig. 1 shows the neutrophil counts in bronchoalveolar washings after oral administration of Compound A at doses of 1 mg/kg, 3 mg/kg and 10 mg/kg in Test Example 1. * indicates that the difference was significant with p < 0.05 as compared with the solvent group by Dunnett's test.

### BEST MODE FOR CARRYING OUT THE INVENTION

Now, the pyridazinone compound represented by the above formula (I) or a pharmaceutically acceptable salt thereof in the antineutrophilia agent of the present invention will be described.

In the formula (I), the C₁₋₆ alkyl groups as R¹, R² and R³ may be linear or branched and may, for example, be methyl, ethyl, n-propyl, isopropyl, n-butyl, isobutyl, sec-butyl, t-butyl, pentyl, hexyl or the like.

R¹ and R² are preferably hydrogen atoms, and R³ is preferably a hydrogen atom or a C₁₋₄ alkyl group.

The C₁₋₄ alkyl group as R³ may, for example, be methyl, ethyl, n-propyl, isopropyl, n-butyl, isobutyl, sec-butyl, t-butyl or the like. Particularly preferred as R³ is a hydrogen atom.

The halogen atoms as X and Y are fluorine atoms, chlorine atoms, bromine atoms or iodine atoms. X is preferably a halogen atom, and Y is preferably a halogen atom or a hydrogen atom.

The C₁₋₈ alkylene which may be substituted with a hydroxyl group as A may be linear or branched and may, for example, be methylene, ethylene, propylene, butylene, pentylene, hexylene, heptylene, octylene, 2,2-dimethylethylene, 2,2-diethylethylene, 2,2-di-n-propylethylene, hydroxymethylene, 1-hydroxyethylene, 2-hydroxyethylene, 3-hydroxypropylene or the like.

A is preferably a C₁₋₅ alkylene which may be substituted with a hydroxyl group.

In the formula (I), the methylene group may be liked to any position in the pyridine ring with no particular restrictions, but preferably is linked to the 3-position to the nitrogen atom in the pyridine ring.

Further, the substituent Y may be at any position in the benzene ring, but preferably at the 4-position.

Pyridazinone compounds of the formula (I) wherein R¹ and R² are hydrogen atoms, R³ is a hydrogen atom or a C₁₋₄ alkyl, X is a halogen atom, Y is a halogen atom or a hydrogen atom, and A is a C₁₋₅ alkylene which may be substituted with a hydroxyl group are particularly preferred.

As preferable compounds, 4-bromo-6-[3-(4-chlorophenyl)propoxy-5-(3-pyridylmethylamino)-3(2H)-pyridazinone, 4-bromo-6-[3-(4-chlorophenyl)-3-hydroxypropoxy]-5-(3-pyridylmethylamino)-3(2H)-pyridazinone and their pharmaceutically acceptable salts are mentioned.

In the present invention, pharmaceutically acceptable salts of the pyridazinone compounds (I) include, for example, salts with inorganic acids (such as hydrochlorides, hydrobromides, phosphates and sulfates), salts with organic acids (such as acetates, succinates, maleates, fumarates, malates and tartrates). These salts may be obtained from the pyridazinone compounds (I) by known methods.

The pyridazinone compounds (I) of the present invention and their pharmaceutically acceptable salts cover their stereoisomers and optical isomers. The pyridazinone compounds (I) and their pharmaceutically acceptable salts are known compounds and known for their low toxicity. They are obtainable, for example, by the methods disclosed in JP-B-7-107055, USP 5314883, EP-A-482208, JP-A-7-252237, USP 5750523 and EP-A-742211.

The pyridazinone compounds (I) of the present invention and their pharmaceutically acceptable salts have excellent antineutrophilia action in mammals such as humans, canines, bovines, equines, rabbits, mice and rats.

The pyridazinone compounds (I) of the present invention and their pharmaceutically acceptable salts are administered at appropriate doses selected depending on the age, weight and conditions of the patient and usually administered to an adult human in an amount of from 0.001 mg to 5 g per day, preferably from 0.005 to 1000 mg per day, in one to several doses a day.

The pyridazinone compounds (I) of the present invention and their pharmaceutically acceptable salts may be administered parenterally in the form of injections (for subcutaneous, intravenous, intramuscular or intraperitoneal injection), ointments, suppositories, aerosols, eye drops or nasal drops, orally in the form of tablets, capsules, granules, pills, powders, lozenges, chewables, syrups, solutions, emulsions or suspensions. Oral administration is preferred.

The pyridazinone compounds (I) of the present invention and their pharmaceutically acceptable salts may be formulated into various dosage forms in accordance with conventional methods commonly employed for preparation of pharmaceuticals.

For example, tablets, capsules, granules, pills, powders, lozenges or chewables for oral administration may be prepared by using an excipient (such as sugar, lactose, glucose, starch or mannitol), a binder (such as syrups, gum Arabic, gelatin, sorbitol, tragacanth, methylcellulose, or polyvinylpyrrolidone), a disintegrant (such as starch, carboxymethylcellulose or its calcium salts, microcrystalline cellulose or polyethylene glycol), a gloss agent (such as talc, magnesium stearate, calcium stearate or silica) or a lubricant (such as sodium laurate or glycerol) by known methods.

In the case of formulations for oral administration, organic acids such as citric acid, succinic acid, maleic acid, fumaric acid, malic acid and tartaric acid may be added to improve solubility and absorbability.

Injections, aerosols, syrups, solutions, emulsions, suspensions, eye drops and nasal drops may be prepared by using a solvent for the active ingredient (such as water, ethyl alcohol, isopropyl alcohol, propylene glycol, 1,3-butylene glycol or polyethylene glycol), a surfactant (such as a sorbitan fatty acid ester, a polyoxyethylene sorbitan fatty acid ester, a polyoxyethylene fatty acid ester, a polyoxyethylene ether of hydrogenated castor oil or lecithin), a suspending agent (such as a cellulose derivative like the carboxymethyl sodium salt or methylcellulose or a natural rubber like tragacanth or gum Arabic) or a preservative (such as a p-hydroxybenzoate ester, benzalkonium chloride or a salt of sorbic acid) by ordinary methods. Suppositories may be prepared by using e.g., cacao butter, polyethylene glycol, lanolin, a fatty acid triglyceride or coconut oil by known methods.

Ointments to be absorbed percutaneously may be prepared by using e.g., white petrolatum, liquid paraffin, higher alcohols, macrogol ointment, a hydrophilic ointment or an aqueous gel base.

### EXAMPLES

Now, the present invention will be described in further detail with reference to Test Examples and Examples. However, it should be understood that the present invention is by no means restricted by these specific Examples.

In the following Test Examples and Examples, Compound A (4-bromo-6-[3-(4-chlorophenyl)propoxy]-5-(3-pyridylmethylamino)-3(2H)-pyridazinone hydrochloride) prepared by an ordinary method was used. The other reagents were purchased.

### [TEST EXAMPLE 1]

### Effect of Compound A on neutrophilia in the rat airway

Male Wister rats weighing from 100 to 200 g were put in a clear plastic inhalation box for small animals (W30×H30×D30 cm), and 30 mL of a lipopolysaccharide solution (LPS E.coli 0.3 mg/mL saline) atomized to an average particle size of 2.0-6.0 µm was administered by inhalation from an ultrasonic nebulizer (TUR-3200, NIHON KOHDEN) for 30 minutes.

The lipopolisaccharide inhalation was preceded, 30 minutes in advance, by oral administration of Compound A suspended in 0.5% methylcellulose (MC) at doses of 1 mg/4 mL/kg, 3 mg/4 mL/kg and 10 mg/4 mL/kg, or by oral administration of 0.5% MC at a dose of 2 mL/kg to a solvent group. To the non-treatment group, 0.5% MC was orally administered at a dose of 2 mL/kg, 30 minutes before physiological saline inhalation. Each group consisted of 6 rats.

About 5 hours after the lipopolisaccharide inhalation, bronchoalveolar washings were collected. Namely, an intraperitoneal urethane injection was given to a rat, and then the airway was irrigated with 5 mL of physiological saline through a tracheal cannule inserted through a cut made in the trachea by repeating infusion and suction twice. The irrigation was repeated twice to collect 10 mL of bronchoalveolar washings. The bronchoalveolar washings were immediately centrifuged at 4°C at 1471 m/s2 for 10 minutes. The cell precipitate was suspended in 0.5 mL of 0.2% physiological saline, and 1 minute later, 1.6% physiological saline was added. The total number of leukocytes in the suspension was counted with a multichannel blood cell counter and designated as the total leukocyte count.

Further, after the cell suspension was adjusted to a total leukocyte count of 1x10⁶ cells/mL, 100 µL of the suspension was centrifuged at room temperature at 400 rpm for 4 minutes in a cytocentrifuge (Thermo Shandon) and made into smears. The smears were stained with Diff-Quick (International Reagents Co., Ltd.), and monocytes, eosinophils and neutrophils were counted under an inverted microscope (x 400) to about 500 cells. The number of each type of leukocytes was calculated from the ratio to the total leukocyte count in accordance with the following formula.

The number of each type of leukocytes = the total leukocyte count x the ratio of each type of leukocytes (the number of the counted leukocyte of each type / the total number of the counted cells)

Statistic analysis was done with the SAS Preclinical Package V5 software. A "t-test between two groups" for analysis of single-factor experimental data was used to determine if there was significant difference between the negative control group and the lipopolysaccharide solvent groups, and "Dunnett's parametric multiple comparison test" for analysis of single-factor experimental data was used to determine if there was significant difference between the control group and the treated groups. The difference between two groups was considered to be significant if p < 0.05 (two-sided).

### [Results]

The results are shown in Fig. 1. It was observed that the endotoxin inhalation induced accumulation of neutrophils in the airway of the Wister rats. Compound A had antineutrophilia effect in the rat airways when orally administered at doses of 1 mg/kg, 3 mg/kg and 10 mg/kg.

### [TEST EXAMPLE 2]

Effect of Compound A in guinea pig respiratory dysfunction models with airway neutrophilia induced by exposure to tobacco smoke

### [Methods]

Hartley guinea pigs weighing from 350 to 450 g were exposed to cigarette smoke for an hour per day, five days per week, for 3 weeks, by using a tobacco smoke exposer and an exposure chamber (Flow-pasttype nose-only inhalation chamber, Muenster).

The tobacco smoke exposure was preceded, 15-45 minutes in advance, by oral administration of the compound suspended in 0.5% methylcellulose (MC) at a dose of 10 mg/2 mL/kg or by oral administration of 0.5% MC at a dose of 2 mL/kg to a solvent group. To the non-treatment group, 0.5% MC was orally administered at a dose of 2 mL/kg, 15-45 minutes before air exposure. From 4 to 6 rats were carried out for each group. The airway resistances and neutrophil counts were measured the day after three weeks of the tobacco smoke exposure. The airway resistances were measured by double chamber plethysmography with a respiratory function tester (Puloms-1, M.I. P. S.) during wakefulness. The neutrophil counts were measured in the same manner as in Test Example 1. The effect of Compound A was evaluated by calculating the suppression ratio (%) in accordance with the following formula.

Suppression ratio = ((measured value for the control group - measured value for the normal value) - (measured value for the Compound A group - measured value for the normal group)) x 100 / (measured value for the control group - measured value for the normal value)

### [Results]

Compound A inhibited neutrophilia by 73% and suppressed increase in airway resistance by 100%.

It is obvious that Compound A is effective against respiratory dysfunction with neutrophilia due to exposure to tobacco smoke in guinea pigs.

### EXAMPLE 1 (Tablets)

10 g of Compound A, 20 g of lactose, 5 g of starch, 0.1 g of magnesium stearate and 7 g of calcium carboxymethylcellulose, 42.1 g in total, were mixed by an ordinary method and made into sugar-coated tablets each containing 50 mg of Compound A.

### EXAMPLE 2 (Tablets)

Tablets containing 10.0 mg of Compound A as the base, 5.0 mg of citric acid as an organic acid, 123.0 mg of lactose as an excipient, 4.0 mg of hydroxypropylcellulose as a binder, 7.0 mg of croscarmellose sodium as a disintegrant and 1.0 mg of magnesium stearate as a gloss agent were prepared.

### EXAMPLE 3 (Capsules)

10 g of Compound A, 20 g of lactose, 10 g of microcrystalline cellulose and 1 g of magnesium stearate, 41 g in total, were mixed by an ordinary method and put in gelatin capsules to obtain capsules each containing 50 mg of Compound A.

### EXAMPLE 4 (Aerosol suspension)

The following ingredients (A) were mixed, and the resulting liquid mixture was loaded into a valved vessel. The propellant (B) was injected through the valve nozzle at 20°C to a gauge pressure of about 2.46 to 2.81 mg/cm² to obtain an aerosol suspension.
(A): Compound A 0.25 mass%, isopropyl myristate 0.10 mass%, ethanol 26.40 mass%
(B): a 60-40 mass% mixture of 1,2-dichlorotetrafluoroethane and 1-chloropentafluoroethane: 73.25 mass%

## Claims

1. An antineutrophilia agent containing a 3(2H)-pyridazinone compound represented by the formula (I) or a pharmaceutically acceptable salt thereof. 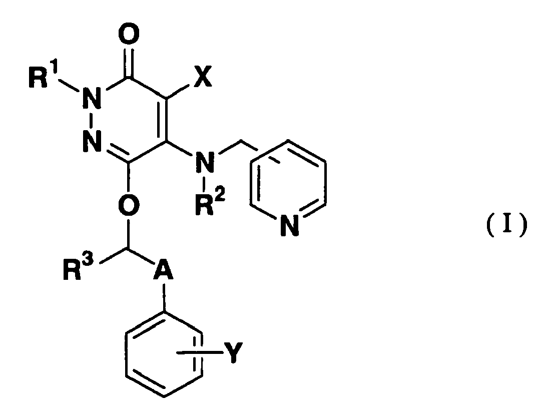 wherein each of R¹, R² and R³ is independently a hydrogen atom or a C₁₋₆ alkyl group, X is a halogen atom, cyano or a hydrogen atom, Y is a halogen atom, trifluoromethyl or a hydrogen atom, and A is a C₁₋₈ alkylene which may be substituted with a hydroxyl group.

2. The antineutrophilia agent according to Claim 1, wherein in the formula (I), R¹ and R² are hydrogen atoms, R³ is a hydrogen atom or a C₁₋₄ alkyl group, X is a halogen atom, Y is a halogen atom or a hydrogen atom, and A is a C₁₋₅ alkylene which may be substituted with a hydroxyl group.

3. The antineutrophilia agent according to Claim 1, wherein the compound represented by the formula (I) is 4-bromo-6-[3-(4-chlorophenyl)propoxy-5-(3-pyridylmethylamino)-3(2H)-pyridazinone or 4-bromo-6-[3-(4-chlorophenyl)-3-hydroxypropoxy]-5-(3-pyridylmethylamino)-3(2H)-pyridazinone.

4. The antineutrophilia agent according to Claim 1, wherein the pharmaceutically acceptable salt is an organic acid salt or an inorganic acid salt.

5. The antineutrophilia agent according to any one of Claims 1 to 4, which is a preventive or therapeutic agent for chronic obstructive pulmonary disease.
